# EUROPEAN PATENT SPECIFICATION

(11) **EP 1 853 227 B1**
(45) Date of publication and mention of the grant of the patent: **05.08.2009**
(21) Application number: 06709975.4
(22) Date of filing: 02.03.2006
(51) Int. Cl.: A61K 9/16, A61K 39/07, A61K 39/00

(54) **PHARMACEUTICAL COMPOSITION**
PHARMAZEUTISCHE ZUSAMMENSETZUNG
COMPOSITION PHARMACEUTIQUE

(30) Priority: 02.03.2005 GB 0504276; 10.06.2005 GB 0511801
(43) Date of publication of application: 14.11.2007
(73) Proprietor: THE SECRETARY OF STATE FOR DEFENCE, Salisbury Wiltshire SP4 0JQ (GB)
(72) Inventor: EYLES, James Edward, Salisbury Wiltshire SP4 0JQ (GB); WESTWOOD, Angela, Salisbury Wilthshire SP4 0JQ (GB); ELVIN, Stephen, J., Salisbury Wiltshire SP4 0JQ (GB); HEALEY, Gareth David, Salisbury Wiltshire SP4 0JQ (GB)
(74) Representative: Farnsworth, Alastair Graham
(86) International application number: PCT/GB2006/000751
(87) International publication number: WO 2006/092607

(56) References cited:
- WO-A-20/04062599
- DIEBOLD S ET AL: "Innate antiviral responses by means of TLR7-mediated recognition of single-stranded RNA" SCIENCE, AMERICAN ASSOCIATION FOR THE ADVANCEMENT OF SCIENCE,, US, vol. 303, 5 March 2004 (2004-03-05), pages 1529-1531, XP002358690 ISSN: 0036-8075
- ELAMANCHILI P ET AL: "Characterization of poly(d,l-lactic-co-glycolic acid) based nanoparticulate system for enhanced delivery of antigens to dendritic cells" VACCINE, BUTTERWORTH SCIENTIFIC. GUILDFORD, GB, vol. 22, no. 19, 23 June 2004 (2004-06-23), pages 2406-2412, XP004515457 ISSN: 0264-410X

## Description

The present invention relates to microparticle and pharmaceutical compositions comprising stable, Immunogenic, microencapsulated single-stranded ribonucleic acid (RNA), which stimulates an immune response in mammalian cells. Methods for the production of such a pharmaceutical composition and uses in immunotherapy are also claimed and described.

The mammalian immune system possesses a specialised 'early warning system' in the form of a panel of detectors to rapidly sense and trigger responses to the presence of microbial invaders. The various 'toll-like receptors' (TLRs) recognise diverse structures and chemicals that are conserved in pathogens but are not found in multicellular organisms. Stimulation of a TLR with its appropriate agonist results in the activation of signalling pathways leading to the production of specific proinflammatory and / or anti-viral cytokine(s). There is now considerable interest in using certain TLR agonists, such as CpG oligodeoxynucleotides, as immunotherapeutics and vaccine adjuvants (see for example Nature Reviews Immunology 2004, Vol. 4 pages 248-257 and pages 512-520).

Amongst infection induced cytokines, type I interferons (IFN-α and IFN-β) are notable for their importance in antiviral responses. Type I interferons are Involved very early on in the innate immune response and are critical to rapidly establishing an antiviral state following infection. Type I interferons also modulate any subsequent adaptive response to virus; encouraging dendritic cell cross priming and the induction of cytotoxic T-lymphocyte (CTL) responses.

Plasmacytoid dendritic cells can produce extremely high levels of type I interferon (up-to 1000 times more than conventional cells). It is thought that plasmacytoid dendritic cells are responsible for systemic interferon responses to many viral infections. Stimulation of plasmacytoid dendritic cells with certain CpG oligonucleotides results in the production of high levels of interferon-a. It is thought that CpG DNA interacts with TLR9 in the endosomal compartment Recently, it has been demonstrated that murine plasmacytoid dendritic cells can produce high levels of IFN-α following stimulation with guanine and Uracil-rich sequences of single stranded (ss) RNA (Diebold et al Science 2004, 303, 1529). There is good evidence that this effect is mediated by TLR7 (in mice). Like TLR9, it is thought that TLR7 interacts with pathogen associated molecular patterns in endosomal compartments. The cellular location of TLR7, i.e. in the endosomal compartment, prevents activation by 'self' ssRNA In the cytosol. However, it is generally accepted that ssRNA alone is unable to effectively stimulate IFN-α production from plasmacytoid dendritic cells. This is because ssRNA is particularly susceptible to nuclease attack, and also because uptake of ss-RNA's across plasma membranes is particularly poor.

There is therefore a need to provide a means of stabilising such ss-RNA molecules such that they are suitable for use in immunotherapy. Such a means for stabilising the ss-RNA should protect the RNA from nuclease attack whilst maintaining its immunostimulatory properties. In addition, the means for stabilising the ss-RNA should Ideally be in a form that is complementary with existing means for delivering immunogenic compounds, such that the generic immune response elicited by ss-RNA does not interfere with any specific therapies that may be administered or co-administered.

The present inventors have now found that certain formulations comprising ss-RNA which has been microencapsulated in the presence of a stabilising agent successfully stabilises and protects ss-RNA and also stimulates an increased cytokine response over other generic therapeutic agents. In particular, the composition of the present invention stimulates levels of IFN-α and IL-12. which are at least equivalent, if not superior, to those stimulated by ss-RNA condensed with commonly utilised *in vitro* transfection reagents (such as poly ethylene imine, hereinafter "PEI"). This type of composition has particular advantages for *in vivo* administration: it is likely to be less toxic than the traditionally-stabilised ssRNA described above because it contains relatively low levels of stabilising agent. Also, the particle size of the microparticulate formulation can be optimised depending on the required route of administration. For example, the microparticles of the present invention may be formed so as to provide an average particle size within the range perceived to be optimal for delivery to the lower respiratory tract, i.e. 1-10µm.

The microparticle and pharmaceutical compositions of the present invention stimulate production of cytokines in a host cell and, as such, may be used as a generic immunotherapy for a wide range of conditions and infections. This is particularly advantageous when a specific therapy is not known or is unavailable. A further advantage of the invention is that the composition optionally comprises specific therapeutic agents such that a generic and a specific immune response may be elicited in a compromised individual to whom the composition is administered. The microparticle composition of the present invention has the added advantage in that it may be stored as a dry powder for extended periods of time (at least several months)without loss of biological potency. After storage, the composition may be administered directly as a dry powder, for example by inhalation, or re-hydrated in a pharmaceutical acceptable solvent or buffer, as are known in the art, and administered parenterally, as required.

The compositions of the present invention may also be useful in eliciting a cytotoxic T-lymphocyte (CTL) response, which is particularly important for the clearance of intracellular pathogens and cancerous cells. This feature of the composition highlights a particular advantage of the invention because, in light of the importance of IFN-α in the induction of CTL responses, it can provide a vaccine delivery system capable of Inducing a CTL response. Such a vaccine delivery system is particularly suitable for tumour immunotherapy.

According to the present invention there is provided a microparticle composition comprising a biodegradable polymer, an immunogenic single-stranded ribonucleic acid (hereinafter "ss-RNA") material, a biologically active macromolecule and a stabilising agent wherein the biologically active macromolecule, the single-stranded ribonucleic acid (ss-RNA) and the stabilising agent are encapsulated inside and/or within the biodegradable polymer to provide a free outer surface of the microparticle. It will be understood by those skilled in the art that whilst the outer surface of the microparticle is essentially free of the entrapped components, it is inevitable that a small proportion of each of the ss-RNA, the biologically active macromolecule and the stabilising agent may be present of the surface of the microparticle. However, as described herein, the outer surface of the microparticles are substantially free of the entrapped components. Therefore, as used herein, "free outer surface" relates to the outer surface of microparticles which comprise no adsorbed components directly after formation. However, microparticles with such free outer surfaces may be subjected to processes which result in other materials, e.g. pharmaceutical compounds, macromolecules and nucleic acids, becoming adsorbed thereto. As used herein "absorbed components'' relates to entrapped materials which may be partially present at the microparticle surface, as a result of the microparticle formation process and "adsorbed components" relates to materials which are adsorbed on to the outer surface of the microparticle, after it has been formed.
As used herein, the term "microparticle" refers to a particle of from about 10 nm to about 100 µm in diameter, preferably in the range of from 200 nm to 30 µm and more preferably in the range of from 500 nm to 10 µm in diameter. Microparticle size is readily determined by techniques well known in the art, such as laser diffractometry or scanning electron microscopy, and is commonly quoted as an average diameter. The terms particle, particulate, microparticles, microparticulate and microsphere may be used interchangeably and all fall within the above definition of microparticle.

Any biodegradable polymer which is blocompatible may be used to form the microparticles of the present invention but it is preferred that a polymer which is known to degrade in mammalian tissue and which is suitable for pharmaceutical administration is used. Examples of such biodegradable polymers include, but are not limited to, aliphatic polyesters, polymers derived from a poly(a-hydroxy acid) such as poly(lactide) ("PLA"), or a copolymer of D, L-lactide and glycolide or glycolic acid, such as poly(D,L-lactide-co-glycolide ("PLG" or "PLGA") or a polyglycolide, a polycaprolactone and copolymers thereof. It is preferred that the biodegradable polymer is a poly(lactide).

The ss-RNA material encapsulated within the microparticles can be any single stranded sequence of RNA which is capable of stimulating or enhancing an immune response, particularly by stimulating production of proinflammatory cytokines, such as Tumour Necrosis Factor (TNF-α) and /or anti-viral cytokines, such as INF-α, INF-β, or IL-12. It is preferred that ss-RNA stimulates the production and secretion of INF-α. It will be understood by those skilled in the art that INF-α may be produced by many mechanisms, but it is preferred that the ss-RNA is chosen such that it stimulates production of the cytokine by interacting with and stimulating Toll-Like Receptors (TLR) of dendritic cells in a mammalian host. It will also be understood by those skilled in the art that different TLRs may facilitate the secretion of INF-α, but it is preferred that the ss-RNA stimulates TLR-7 and/or TLR-8, which are likely to be involved in cytokine expression in humans.

Suitable ss-RNA sequences are those which have a high proportion of a single base, that is, sequences which contain regions which are rich in either A, U, C or G. It is preferred that the ss-RNA has a G-rich or a U-rich sequence. Preferred examples of ss-RNA include polyuridylic acid and polyguanylic acid. It is more preferred that the ss-RNA is polyuridylic acid.

Microencapsulation of the ss-RNA is preferably performed in the presence of a biologically active macromolecule. Such macromolecules include therapeutic agents known to elicit generic immune responses, such as a oligodeoxynucleotides, CpG oligonucleotides, polypeptides and proteins and also substances capable of providing a specific therapeutic effect, such as agents which elicit a specific immune effect against a known pathogen, such as a pathogen-specific antigen or, in the alternative, substances which elicit a specific immune effect against an antigen expressed on a tumour or cancerous cell.

As used herein, the term "antigen" means a molecule which contains one or more epitopes capable of stimulating a host's immune system to make an antigen-specific immune response when the antigen is present or to elicit a humoral antibody response. The term antigen denotes both sub-unit antigens and killed, attenuated or inactivated bacteria, viruses and other microbes. Antibodies and fragments of antibodies which can mimic an antigen or an antigenic determinant are also include in the definition of antigen.

In a preferred embodiment, the microparticle composition comprises an antigen specific to a bacterial pathogen, for example recombinant Protective Antigen (rPA) of *Bacillus anthracis* or F1 and or V antigens from *Yersinia pestis* but it will be understood by those skilled in the art that any known antigen may be formulated into the microparticle composition. This embodiment is particularly useful for the treatment of bacterial infections, such as anthrax, plague, melioidosis, glanders, chlamydia and the like but also for the treatment of viral, fungal and parasitic infections, provided a suitable antigen or antigen-mimic is available.

The stabilising agent of the microparticle formulation can be anyone selected from the wide range of stabilising agents known in the technical field. Stabilising agents include but are not limited to detergents, surfactants, dispersing agents, suspending agents and emulsion stabilisers. Preferred examples of stabilising agents include lipids and surfactants. However, it is preferred that the stabilising agent selected is a pharmaceutically acceptable agent, such that stabilising agents which may be toxic when administered in-vivo can be avoided. It is further preferred that the stabilising agent is capable of forming a complex with the ss-RNA. Such complexes may be formed by direct covalent attachment of the stabilising agent to the ss-RNA, for example as a result of a condensation reaction or alternatively the complexes may be formed by electrostatic, ionic or hydrophobic interactions. It is more preferred that the stabilising agent is positively charged, so as to effect an electrostatic interaction with the ss-RNA. Suitable examples of such stabilising agents are cationic polymers and/or cationic lipids. Preferred examples are cationic lipids such as cetyl trimethyl ammonium bromide (hereinafter "CTAB"), dimethyl dioctodecyl ammonium bromide ("DOA") and N-[1-(2,3-dioleoyloxy)propyl]-N,N,N-trimethylammonium chloride ("DOTAP") and the like.

Suitable amounts of stabilising agent can be routinely determined by those skilled in the art, but it is preferred that the mass ratio of ss-RNA to biodegradable polymer to stabilising agent is in the range of from about 1:8:6 to about 1:15:12 and more preferably the mass ratio of components is approximately 2:25:18.

The inventors have found that the selection and amount of stabilising agent may affect the overall charge of the microparticles formed in the presence of said stabilising agents such that the pharmaceutical composition may have a net negative or positive charge. It is preferred that the composition has a net positive charge, as measured by a suitable charge-measuring devices such as a Zetasizer™, which measures zeta potential, the electrical potential that exists across the interface of all solids and liquids. It is further preferred that the measured zeta potential of the resulting composition is in the range of from about 0 to 100 mV, more preferably in the range of 20 to 80 mV and even more preferably in the range of 30 to 60 mV. The inventors have found that pharmaceutical compositions with zeta potential of around 50 mV are particularly effective in stimulating production of INF-α.

The microparticle composition ideally comprises microparticles that are of a size which enables efficient administration and transport into the immune system, and which are particularly suitable for inhalational administration. It is preferred that the microparticles have a mean diameter in the range of 0.1 to 5µm, more preferably 0.2 to 4 µm. It is most preferred that the microparticles have a mean diameter of approximately 1 µm. Such microparticles are suitably formulated into a pharmaceutical composition by combining the microparticles with pharmaceutically acceptable adjuvants and/or excipients, such as binders, fillers, diluents, lubricants, colours, sweeteners, dispersing agents and the like.

Pharmaceutical compositions may be formulated to provide a means of co-administering the microparticles with another therapeutic agent or adjuvant. The free outer surface of the microparticles provide a convenient location for the adsorption of further therapeutic agents, such as additional antigens, immunogenic proteins and polypeptides, nucleic acids such as CpG oligonucleotides and DNA vectors. Such additional therapeutic agents can be used to decorate the free outer surface of the microparticles to provide an enhanced immune response.

According to a second aspect of this invention, there is provided a method of producing such microparticle and pharmaceutical compositions, said method comprising the steps of:
(a) Preparing a solution of biodegradable polymer
(b) adding to the solution of (a), a solution comprising an immunogenic ss-RNA and a biologically active macromolecule, to form an emulsion
(c) adding the emulsion from step (b) to a solution containing the stabilising agent to form a double emulsion;
(d) removing the solvent; and
(e) collecting the resulting microparticles (a)

The ss-RNA for use in the method is selected from the list already described herein and is conveniently dissolved in an aqueous solution, for example distilled water or aqueous buffer, and the biodegradable polymer dissolved in a water immiscible solvent, such as an organic solvent, e.g. dichloromethane, but it will be understood by those skilled In the art that any combination of solvents may be used provided that they are capable of forming an emulsion when mixed. The biodegradable polymer is conveniently selected from the list of alternatives already provided herein. Again it will be understood that the stabilising agent may be selected from those stabilising agents already described above may be dissolved in any solvent such that a double-emulsion is formed when the solution of the stabilising agent is added to the first emulsion, that is, the emulsion created in step (b).. Thus, it is preferred that an aqueous or water-miscible solvent is used to dissolve the stabilising agent, in step (c). Such a combination will produce a water-oil-water (w-o-w) double emulsion but it will be understood that an oil-water-oil (o-w-o) double emulsion may be equally suitable, the first emulsion, that is, the emulsion created by step (b) is added to the solution of the stabilising agent in step (c). Although it is preferred that the addition occurs in this order, it is possible to form a suitable double emulsion (and hence microparticles) by add the solution of stabilising agent to the emulsion of step (b). It is preferred that the emulsion from step (b) is added to the solution of the stabilising agent with mixing since the inventors have found that this provides better resulting microparticles. It is more preferred that the emulsion from step (b) is added drop-wise to the solution of the stabilising agent with vigorous mixing. The solvent removal step (d) may be effected by any conventional means, such as constant stirring, vacuum or heat evaporation and the resulting microparticles collected by, for example, filtration or centrifugation. It is more preferred that the microparticles are collected by ultracentrifugation. The microparticles may then be collected and used directly or subjected to further treatments, processing or formulation, such as combining with pharmaceutically acceptable compounds to form a pharmaceutical composition. Although further processing may be desirable for some applications, it may, inmost cases, be unnecessary as the resulting microparticles are of a size which are efficacious when used without further processing.

Microparticle compositions prepared according to the method of the present invention may be further subjected to a lypophilisation step, such that they can be stored for extended periods as a dry powder. The inventors have found that such lyophilised compositions are stable for several months and have the advantage that they can be used directly as dry powder, for mucosal administration via, for example, a conventional inhaler device. Alternatively the dry powders may be rehydrated as and when required, which is particularly advantageous for the preparation of compositions which are suitable for parenteral administration.

The Invention will now be particularly described by way of example, with reference to the accompanying diagrammatic drawings which are described as follows:

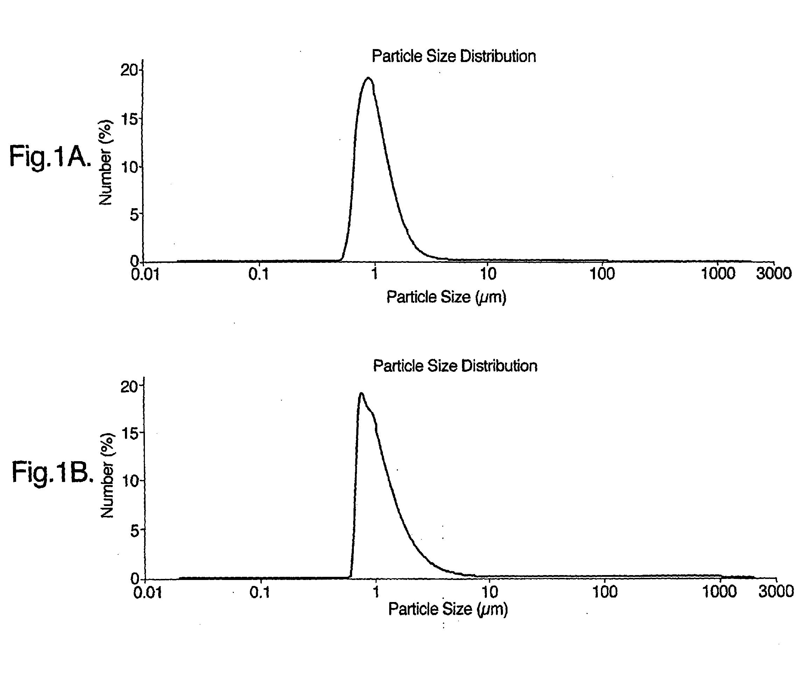

**Figure 1** shows particle size distribution graphs of RNA loaded microparticles, prepared using polyvinyl alcohol (PVA) or N-[1-(2,3-Dioleoyloxy)propyl]-N,N,N-trimethylammonium chloride (DOTAP) as stabilising agents, as determined using laser diffraction measurements. Data are representative of three separate experiments.

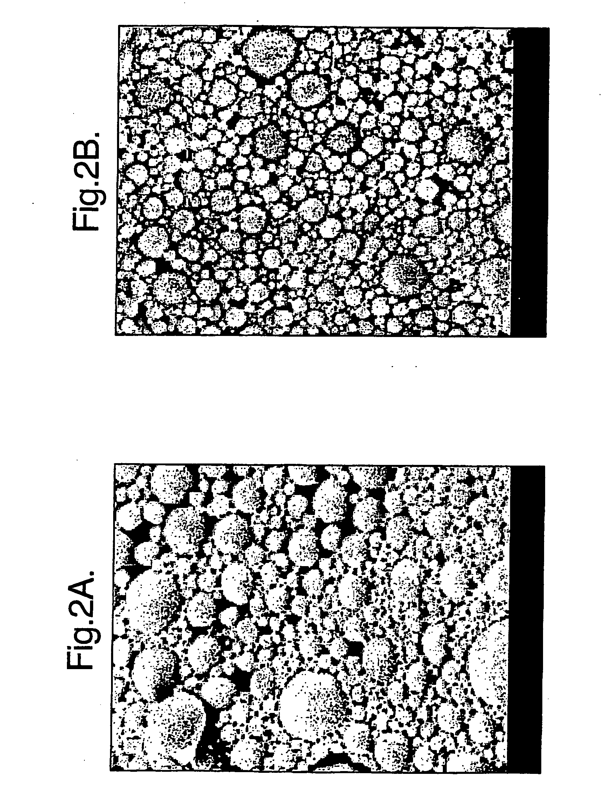

**Figure 2** shows scanning electron micrographs of poly-U loaded polylactide microparticles prepared using polyvinyl alcohol (PVA) or N-[1-(2,3-Dioleoyloxy)propyl]-N,N,N-trimethylammonium chloride (DOTAP) as stabilising agents.

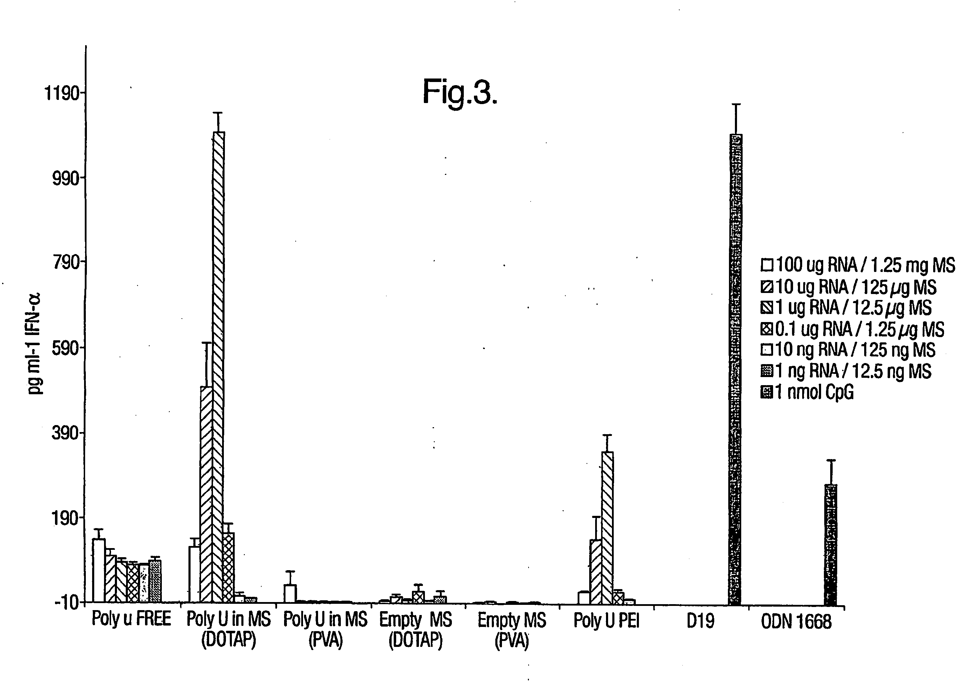

**Figure 3** shows interferon-alpha levels in supernatants from bulk cultures of Fit3L expanded bone marrow derived dendritic cells following overnight stimulation with a range of concentrations of poly-U in solution (Poly-U FREE), poly-U encapsulated in polylactide microparticles (Poly-U in MS), empty polylactide microparticles (Empty MS) and poly-U condensed with PEI (Poly-U PEI). Polylactide microparticles were prepared using either N-[1-(2,3-Dioleoyloxy)propyl]-N,N,N-trimethylammonium chloride (DOTAP) or polyvinyl alcohol (PVA) as a stabiliser. For comparison, cells were also cocultured with 1 nmol CpG. Data are means (±SD) of triplicate microcultures and are representative of three separate experiments.

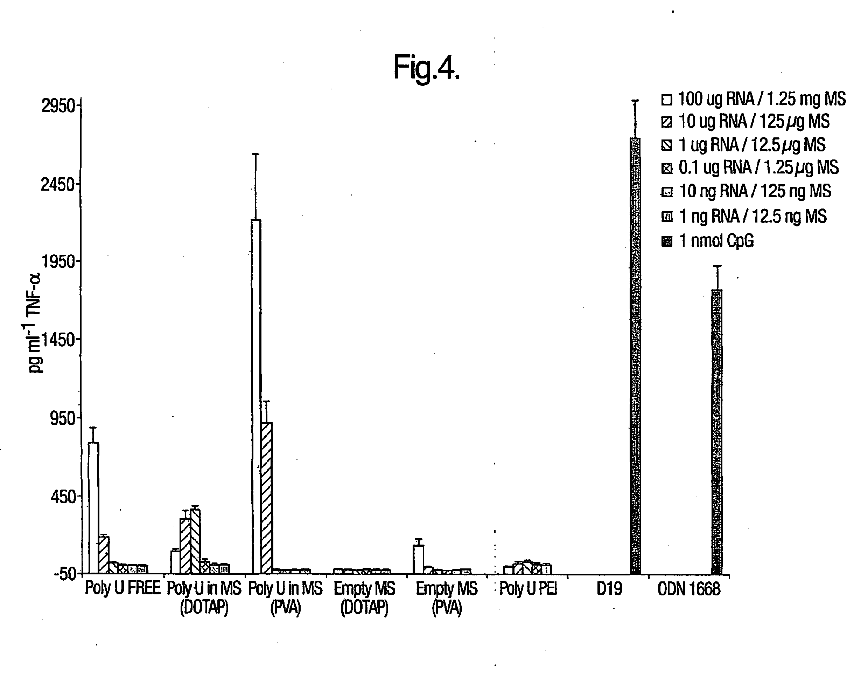

**Figure 4** shows TNF-alpha levels in supernatants from bulk cultures of FIt3L expanded bone marrow derived dendritic cells following overnight stimulation with a range of concentrations of poly-U in solution (Poly-U FREE), poly-U encapsulated in polylactide microparticles (Poly-U in MS), empty polylactide microparticles (Empty MS) and poly-U condensed with PEI (Poly-U PEI). Polylactide microparticles were prepared using either N-[1-(2,3-Dioleoyloxy)propyl]-N,N,N-trimethylammonium chloride (DOTAP) or polyvinyl alcohol (PVA) as a stabiliser. For comparison, cells were also cocultured with 1 nmol CpG. Data are means (±SD) of triplicate microcultures and are representative of three separate experiments.

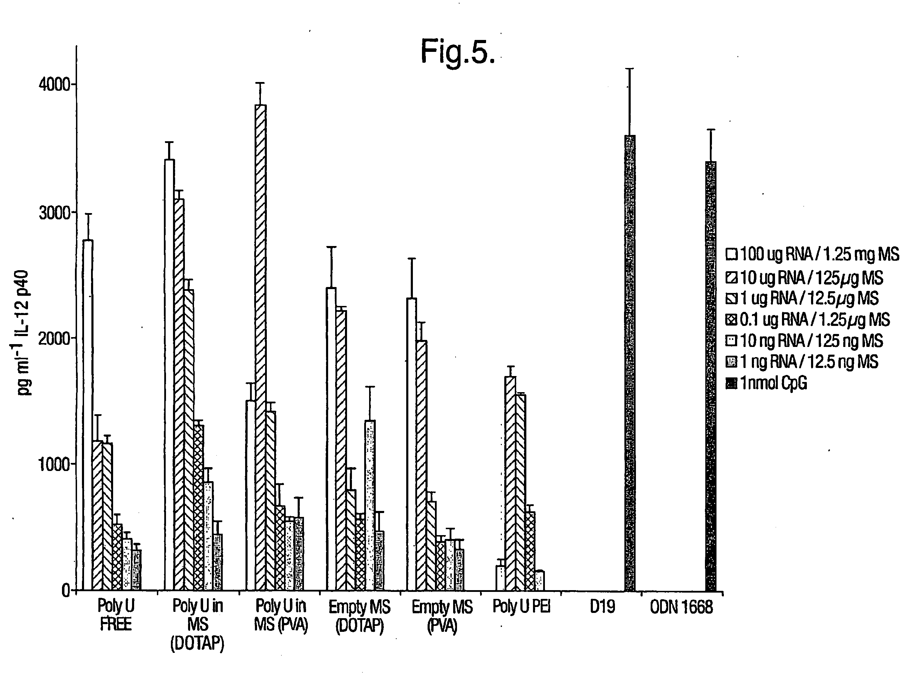

**Figure 5** shows IL-12 p40 levels in supernatants from bulk cultures of FIt3L expanded bone marrow derived dendritic cells following overnight stimulation with a range of concentrations of poly-U in solution (Poly-U FREE), poly-U encapsulated in polylactide microparticles (Poly-U in MS), empty polylactide microparticles (Empty MS) and poly-U condensed with PEI (Poly-U PEI). Polylactide microparticles were prepared using either N-[1-(2,3-Dioleoyloxy)propyl]-N,N,N-trimethylammonium chloride (DOTAP) or polyvinyl alcohol (PVA) as a stabiliser. For comparison, cells were also cocultured with 1 nmol CpG. Data are means (±SD) of triplicate microcultures and are representative of three separate experiments.

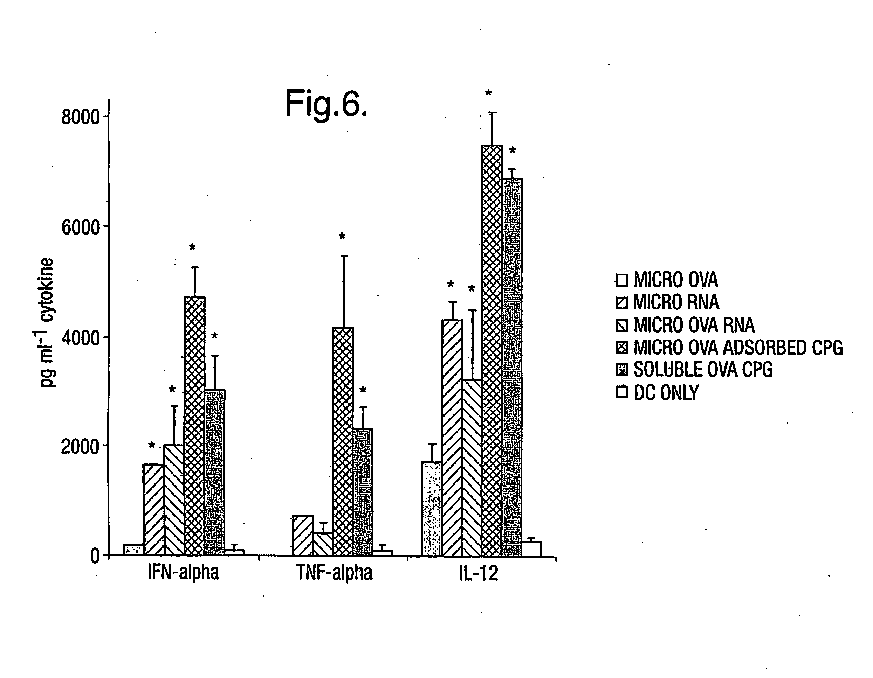

**Figure 6** shows cytokine secretion following overnight stimulation of Flt-3L expanded bone marrow dedritic cells (BMDC) with microencapsulated OVA, microencapsulated poly-U, comicroencapsulated OVA and poly-U, microencapsulated OVA with surface adsorbed CpG, CpG and OVA in solution or media only. Results are means (±SD) of three separate experiments. * denotes statistically significant differences (P<0.05) as compared with microencapsulated OVA treatment.

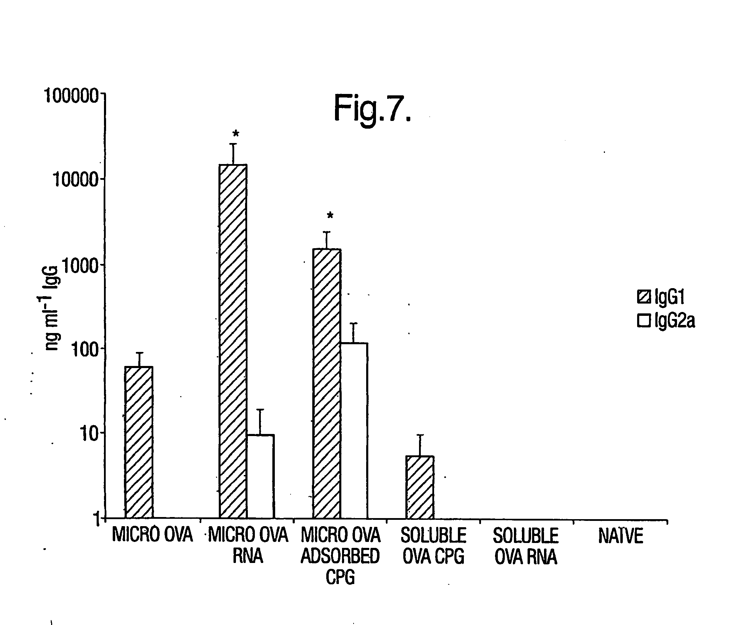

**Figure 7** shows serum anti-OVA IgG titres following subcutaneous immunisation of BALB/c mice with microencapsulated OVA, comicroencapsulated OVA and poly-U, microencapsulated OVA with surface adsorbed CpG, CpG and OVA in solution or poly-U and OVA in solution. Results are means (±SD) with six mice per treatment group. * denotes statistically significant differences (P<0.05) as compared with naive controls.

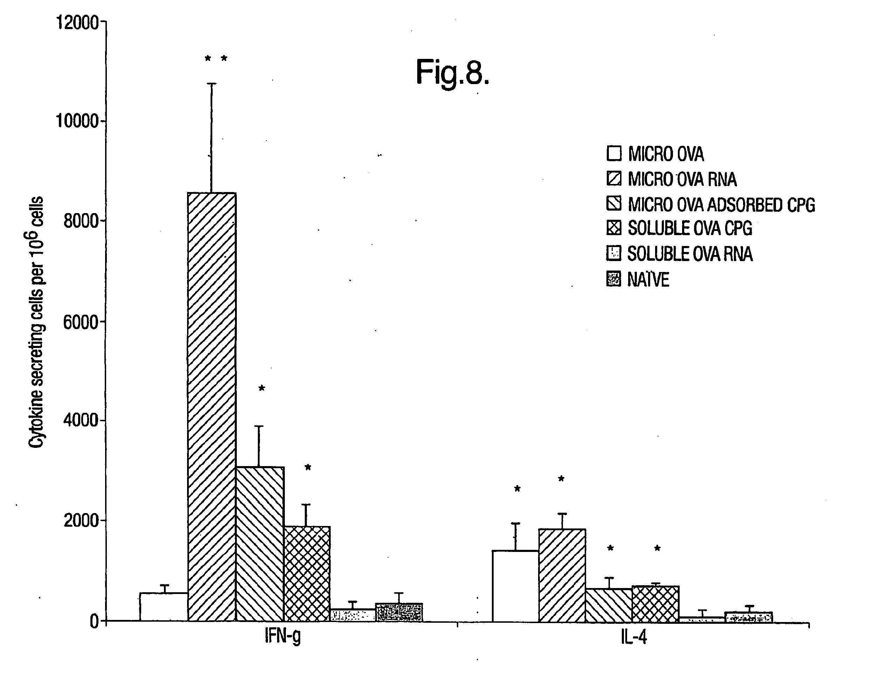

**Figure 8** shows OVA specific IFN-γ and IL-4 ELISPOTS following subcutaneous immunisation of BALB/c mice with microencapsulated OVA, comicroencapsulated OVA and poly-U, microencapsulated OVA with surface adsorbed CpG, CpG and OVA in solution or poly-U and OVA in solution. Results are means (±SD) with six mice per treatment group. * denotes significant differences (P<0.05) as compared with naive controls. ** denotes significant difference as compared with all other treatments (P<0.05).

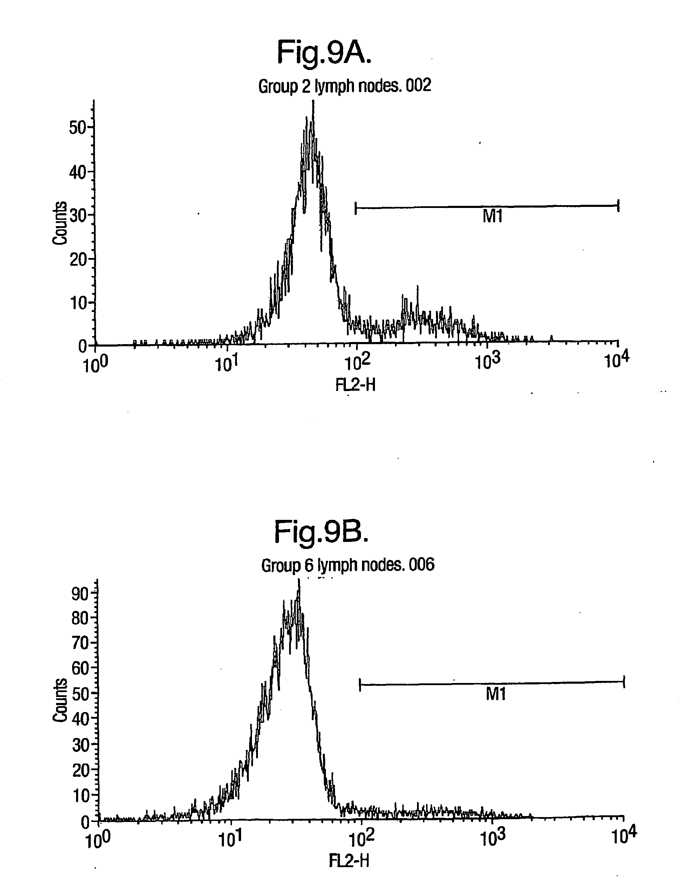

**Figure 9** shows FACS analysis of tetramer stained lymph node cells pooled from six mice immunised with comicroencapsulated OVA and poly-U (top pannel) or naive mice (lower panel). Mice immunised with comicroencapsulated OVA and poly-U had a greater number of CD8 cells with bound tetramer, as evidenced by an increased FL2 signal.

### Example 1:

### Encapsulation of poly-U in polyvinyl alcohol stabilised polylactide microparticles.

Polyuridylic acid (poly-U) (Sigma, Dorset UK) was encapsulated in poly-lactide microparticles. Briefly, 10 mg of poly-U was suspended in 0.5 ml of an aqueous solution of polyvinyl alcohol (PVA) 13-23kDa (1.5 % w/v) (Sigma, Dorset UK) and vigorously mixed with 125 mg of polylactide (PLA) dissolved in 9 ml of dlcholoromethane (DCM) (Sigma, Dorset UK) using a Silverson homogeniser (Silverson, Bucks. UK). The resultant emulsion was added, drop-wise, into a vigorously stirred secondary aqueous phase (90 ml) containing 3.0 % w/v PVA 13-23kDa. Following solvent evaporation, hardened polymeric microparticles were harvested by ultracentrifugation prior to lyophilisation (Edwards, Crawley UK).

### Example 2:

### Encapsulation of poly-U in N-[1-(2,3-Dioleoyloxy)propyl]-N,N,N-trimethylammonium chloride (DOTAP) stabilised polylactide microparticles.

10 mg of poly-U was dissolved in a 0.5ml volume of distilled water. This was vigorously mixed with 125 mg of PLA dissolved in 9 ml of DCM (Sigma, Dorset UK) using a Silverson homogeniser (Silverson, Bucks. UK). The resultant emulsion was added, drop-wise, into a vigorously stirred secondary aqueous phase (90 ml) containing 0.1% w/v DOTAP. Following solvent evaporation, hardened polymeric microparticles were harvested by ultracentrifugation prior to lyophilisation (Edwards, Crawley UK) in 1% w/v trehalose (Sigma, Dorset UK).

### Example 3:

### Encapsulation of poly-U and Ovalbumin (OVA) in DOTAP-stabilised polylactide microparticles.

Ovalbumin (OVA) (Sigma, Dorset, UK) was encapsulated in polylactide microparticles as described In example 2, by co-addition of 5 mg OVA and 10 mg Pofy-U in 0.5 ml of distilled water.

In the above examples, RNA was successfully extracted from both PVA- and DOTAP-stabilised microparticles, as confirmed by NanoDrop technology (results not shown).

### Example 4:

### Preparation of Ovalbumin (OVA) microparticles.

Microencapsulated OVA was prepared as described in example 3 in the absence of poly-U.

### Example 5:

### Adsorption of poly-U to the surface of N-[1-(2,3-Dioleoyloxy)propyl]-N,N,N-trimethylammonium chloride stabilised polylactide microparticles.

Cationic microparticles were prepared using a modified single emulsion solvent evaporation method. 125 mg of PLA dissolved in 9 ml of DCM (Sigma, Dorset UK) was vigorously mixed with a 90 ml volume of 0.1% w/v DOTAP using a Sliverson homogeniser (Silverson, Bucks. UK). Following solvent evaporation, hardened polymeric microparticles were harvested by ultracentrifugation prior to lyophilisation (Edwards, Crawley UK). Poly-U was subsequently adsorbed to the microparticles at a w/w loading of 5% just prior to use.

### Example 6:

### Adsorption of CpG DNA to the surface of OVA loaded microparticles.

Ovalbumin loaded microparticles, prepared as described in example 3, were 'decorated' with CpG (MWG-BIOTECH Ltd, Bucks, UK) by adsorbing CpG to the surface of the microparticles. 7 mg of OVA loaded microparticles were suspended in 0.7 ml of a 800 µg ml-1 solution of CpG DNA (ggTGCATCGATGCAgggggG) in sterile saline. Particles were incubated in this solution for 20 minutes at room temperature.

### Example 7:

### Determination of microparticle size distribution using laser diffraction analysis.

Microparticles prepared as described above in Examples 1, 2, 3 and 4 were sized using a Mastersizer 2000 (Malvem Instruments, Malvern, UK). Laser diffraction measurements revealed that loaded polylactide microparticles had a size distribution of around 1 µm, as shown in Figure 1. Both PVA stabilised (Fig. 1A) and DOTAP stabilised (Fig. 1B) formulations had similar size distributions.

### Example 8:

### Scanning electron microscopy.

Scanning electron microscopy (Hitachi S800) was used to investigate microsphere size and morphology. Images were analysed using Pro Plus image analysis software. Figure 2 shows that both PVA stabilised (Fig. 2A) and DOTAP stabilised (Fig. 2B) formulations had similar size distributions and morphology.

### Example 9:

### Analysis of microparticle surface charge.

Zeta potential measurements of microparticles, prepared in accordance with Examples 1, 2, 3 and 4 were determined using a Zetamaster (Malvern Instruments, Malvern, UK). The results are summarised in Table 1, below, which shows that microparticles stabilised with DOTAP had a significantly more positive zeta potential (+41.81) when compared with microspheres prepared with PVA as a stabiliser (-0.90).

**Table 1. Zeta potential measurements of empty and RNA loaded microparticles. prepared using polyvinyl alcohol (PVA) or N-[1-(2,3-Dioleoyloxy)propyl]-N,N,N-trimethylammonium chloride (DOTAP) as stabilising agents (number of replicates = 3).**

| Formulation | Zeta potential (Mean±SE) |
|---|---|
| PVA Stabilised microparticles containing RNA | -0.90±1.03 |
| Empty PVA-stabilised microparticles | -5.57±1.48 |
| DOTAP stabilised microparticles containing RNA | +41.81±3.77 |
| Empty DOTAP-stabilised microparticles | +46.54±4.78 |

### Example 10:

### Complexation of poly-U with polyethylenimine (PEI).

20µg poly-U was admixed with 30µl 20mM Polyethylenimine (2 kD PEI) (Aldrich) in 150mM NaCl Immediately prior to use in comparison studies with microencapsulated poly-U, prepared as described above In Examples 1, 2 and 5.

### Example 11:

### Isolation and culture of Flt3-L expanded bone marrow derived dendritic cells.

Bulk cultures containing bone marrow derived plasmacytoid and myeloid dendritic cells (BMDCs) were generated using a method adapted from Gillet et al. Journal of Experimental Medicine, 195, (2002) 953. Briefly, 6-8 week old female C57/BL6 (Charles River, UK) were killed by cervical dislocation (in accordance with the Animal (Scientific Procedures) Act 1986). Tibiae and fibulas were removed from the rear legs and then placed in sterile culture media (RPMI-1640) (Sigma, UK) supplemented with 10% heat inactivated foetal bovine serum (FBS) (Sigma, UK); 1% penicillin / streptomycin / glutamine (Sigma, UK) and 50µM 2-Mercaptoethanol (2-ME) (Sigma, UK) for transport to a class II microbiological safety cabinet. A 25-guage needle was then used to flush supplemented culture media through the bone shafts to eject the bone marrow. Cells were washed, then resuspended in 1mL of culture media and a viable cell count determined. Cell concentration was adjusted to 2 x 10⁶.mL⁻¹ and the media further supplemented with 100ng.mL⁻¹ murine Fms-like tyrosine kinase receptor-3 ligand factor (Flt3L) (R&D Systems, Oxford, UK). The cells were plated out using 6-well tissue culture plates (Sterilin, Stone, UK) and incubated at 37°C in a fully humidified environment in the presence of 5% CO₂. After 5 days, half of the medium was removed and replaced with fresh Flt3L supplemented media. After 10 days, cells were washed and re-seeded at 2x 10⁶.mL⁻¹ in sterile flat-bottomed 96-well tissue culture plates (Sterilin, Stone, UK).

### Example 12:

### Dendritic cell activation assays: Poly-U loaded microspheres

Bulk cultures of C57BL/6 Flt3L BMDCs, prepared as described in example 11, were cocultured with escalating doses of poly-U In sterile 96 well flat-bottomed plates. DCs were also cocultured with escalating doses of polylactide microparticle encapsulated ssRNA, as described in Examples 1 and 2. The mass of microencapsulated poly-U added to the cultures was comparable to the doses of free ssRNA used (based on a 100 % theoretical loading efficiency; i.e. an assumption that 100 % of the ssRNA used in the formulation process was incorporated). The effect of incubating the cells with escalating amounts of empty polylactide microparticles was also tested. DCs were also cocultured with escalating doses of PEI condensed ssRNA, prepared as described in Example 10. As a positive control, 1nmol of a K- type (conventional) CpG (ODN1668: tccatgacgttcctgatgct) and a A/D-type CpG (D19: ggTGCATCGATGCAgggggG) were used. Cells were cocultured with the various stimulents for 18 hours. Culture supernatants were obtained by centrifugation of the cells and formulations for 10 minutes at 10000 rpm. DC cultures stimulated with poly U and CpG were quantified using commercially available ELISA kits (R&D systems, Oxford UK).

### Results:

The magnitude of cytokine secretion by bulk cultures of FIt3L expanded borne marrow derived dendritic cells differed according to the nature and dose of stimulant As expected, CpG DNA stimulated the production of high levels of IFN-α as shown in Figure 3. More recently, Diebold et al [Science, 303, (2004), 1529] have documented that PEI complexed poly-U can be used to efficiently stimulate plasmacytoid dendritic cells to secrete type I interferons. The results presented in Figure 3 corroborate this, but also indicate that appropriately formulated polymeric (polylactide) microparticles, containing ssRNA, can be used to stimulate IFN-α secretion from plasmacytoid dendritic cells. To this end, poly-U loaded polylactide microparticles, prepared using DOTAP as a stabilising agent, were potent stimulators of IFN-α production. The levels of IFN-α stimulated by the poly-U loaded DOTAP stabilised microparticles was significantly (P<0.001) higher as compared with DC exposed to un-formulated (free) poly U and comparable with that engendered by stimulation with PEI-condensed poly-U. In contrast, coculture of DC with poly-U loaded polylactide microparticles, prepared using PVA as a stabilising agent, resulted in low levels of IFN-α production. Coculture of DC with 'empty' polylactide microparticles failed to stimulate IFN-α production, irrespective of the type of stabiliser used in the formulation process. Furthermore, stimulation of DC with positively charged polylactide microparticles surface loaded with poly-U was ineffective in terms of stimulating IFN-α production by DC.

Free poly-U and polylactide microparticle encapsulated poly-U, stimulated TNF-α production by bulk cultures of FIt3L expanded bone marrow derived dendritic cells as shown in Figure 4. Highest levels of TNF-α were induced by stimulation with poly-U loaded polylactide microparticles, prepared using PVA as a stabilising agent. By comparison, poly-U loaded polylactide microparticles, prepared using DOTAP as a stabilising agent, were less efficient stimulators of TNF-α production. Poly-U condensed with PEI failed to elicit the production of significant levels of TNF-α.

Production of IL-12p40 by bulk cultures of FIt3L expanded bone marrow derived dendritic cells occurred following a wide range of stimulus as shown in Figure 5. Free poly U was effective, at high stimulatory doses (1-100 µg), of inducing secretion of appreciable levels of IL-12p40. However, at lower stimulatory doses (1-100 ng) poly-U encapsulated In DOTAP stabilised microparticles was the most potent stimulator of IL 12p40 production (P<0.05 as compared with all other poly U treatment groups).

### Example 13:

### Dendritic Cell Activation Assays: OVA-loaded microparticles.

Bulk cultures of C57BU6 Flt3L expanded bone marrow-derived dendritic cells (BMDC) were prepared as described in example 11 and were cocultured with microparticulate formulations containing OVA or OVA coencapsulated with 1 µg poly U. Cells were also stimulated with OVA loaded microparticles decorated with CpG and solutions of OVA and CpG. Protein and nucleic acid loading values were assumed to be 100 % that of the maximum theoretical value. Thus cells were stimulated with a dosage level equivalent to the maximum amount of OVA and CpG that would theoretically be present in the equivalent microparticle treatment group had the encapsulation / adsorption process been 100 % efficient. The effect of incubating the cells with equivalent amounts of empty polylactide microparticles was also tested. Cells were cocultured with the various stimulants for 18 hours. Culture supernatants were obtained by centrifugation of the cells and formulations for 10 minutes at 10000 rpm. Cytokine levels in the supernatants were quantified using commercially available ELISA kits (R&D systems, Oxford UK). Statistical differences were established using ANOVA and Student-Newman-Keuls tests.

### Results:

Bulk cultures of FIt3L expanded BMDC contained both CD11b^{Hi} B220^{Low} (myeloid) and CD11b^{Low} B220^{Hi} (plasmacytoid) type DC, as established using flow cytometry (not shown). The magnitude and pattern of cytokine secretion by bulk cultures of FIt3L expanded BMDC differed according to the nature of stimulant. Figure 6 shows that OVA admixed with CpG DNA stimulated the production of high levels of Interferon-alpha IFN-α. However, IFN-α secretion was greater still when cells were stimulated with OVA loaded microparticles decorated with CpG (P<0.05). Microparticles containing poly-U or poly-U and OVA stimulated higher levels of IFN-α. as compared with empty microparticles or microparticles loaded with OVA alone (P<0.05).

Levels of TNF-α in supernatants was highest when cells were stimulated with OVA loaded CpG decorated microparticles (P<0.05). Stimulation of cells with soluble antigen and CpG also resulted in significant TNF-α production relative to other treatments (P<0.05).

IL-12p40 concentration in culture supernatants was greatest when BMDC were stimulated with OVA loaded microparticles surface decorated with CpG, or CpG and OVA in solution. In comparison to cultures stimulated with OVA loaded microparticles, stimulation with microparticles containing poly-U (with or without OVA) caused enhanced levels of IL-12 p40 secretion (P<0.05).

Stimulation of bulk cultures of FIt3L expanded BMDC with 'naked' poly-U in solution induced negligible quantities of IFN-α TNF-α and IL-12 p40 (results not shown). Poly-U 'decorated' microparticles also failed to stimulate production of IFN-α or IL-12 p40 at all concentrations tested (10µg - 0.001 ng).

### Example 14:

### In vivo immunisation studies.

All experimentation strictly adhered to the 1986 Scientific Procedures Act. 6-8 week old female C57/BL6 (Charles River, UK) mice were immunised by subcutaneous injection into the flank region on days 0, 14 and 28 of the experiment. Microparticle loading values were assumed to be 100 % that of the maximum theoretical value. Hence, mice dosed with soluble antigen / TLR agonist received either the same, or higher, doses as compared with mice immunised with microparticulate material.

Mice received injections of 100 µl sterile saline containing either: (1) 1 mg of Microparticles containing 40 µg of OVA, (2) 1 mg of Microparticles containing 40 µg of OVA and 80 µg poly-U, (3) 1 mg of Microparticles containing 40 µg of OVA and 80 µg of surface adsorbed CpG, (4) a solution containing 40 µg of OVA and 80 µg of CpG, (5) a solution containing 40 µg of OVA and 80 µg of poly U. A sixth group acted as naive controls.

### Example 15:

### Determination of serum anti-OVA antibody levels

Mice immunised according to example 14 were bled on day 32 to obtain serum. Serum was analyzed for anti-OVA antibodies using standard EUSA methodology. Briefly, individual serum samples were aliqoted to microtitre plates pre-coated with OVA (5 µg ml⁻¹ in PBS). Binding of serum antibody was detected with peroxidase-labelled secondary antibody to mouse IgG1 and IgG2a (Harlan-SeraLab; Crawley Down, UK). As each subclass specific conjugate may not be equally reactive with its subclass molecule, to facilitate a comparison of one subclass titer with another, standard solutions (Harlan-SeraLab, Crawley Down, UK) of each subclass antibody in the range of 0.2-50.0 ng ml⁻¹ were assayed. The standard curves generated enabled determination of the mean concentration of each IgG subclass in serum derived from the various treatment groups. A statistical test (Dunnett) was used to establish if any of the immunisation treatments had stimulated higher levels of specific antibody as compared with control (naive) animals.

### Results

By comparison to naive mice, mice injected with OVA loaded microparticles decorated with CpG had significant anti-OVA antibody titres but mice injected with microparticles containing OVA and poly U showed an amplified antibody response, as shown in Figure 7. In the seroconverted mice, IgG1 was the dominant anti-0VA antibody detected. Injection of mice with OVA loaded microparticles or soluble OVA admixed with CpG served to elicit a specific serum anti-OVA igG1 response in some mice, although within group variation entailed that the effect was not statistically significant as compared with naive animals. Injection of a solution of OVA admixed with poly-U induced negligible levels of serum anti-OVA IgG. Anti-OVA IgG2a was detected only in mice that had been immunised with antigen co-formulated with CpG or poly U.

### Example 16:

### Analysis of cellular responses: ELISPOT Analysis

On day 35, mice immunised according to example 14 were killed and individual (not pooled) spleens removed. Single cell suspensions were prepared in supplemented RPMI-1640.

IFN-γ and IL-4 ELISPOT kits (BD Biosciences, Oxford UK) were used according to the manufacturer's guidelines. In brief, 96-well nitrocellulose bottomed-plates were coated with 100µl of 5 µg ml⁻¹ capture antibody in PBS and incubated overnight at 4°C. Free binding sites were blocked with 200µl of supplemented RPMI for 2 hours. Spleen cell concentrations were adjusted to 2.5 x10⁶ cells ml⁻¹ and added to the appropriated well. Analyses were always conducted on cells from individual mice in each treatment group. Cells were stimulated in triplicate with either 5 µg ml⁻¹ OVA in supplemented RPMI 1640, supplemented RPMI 1640 alone as a negative control or 2.5 µg ml⁻¹ Concanavalin A (Sigma, Dorset, UK) as a positive control overnight. The cells were removed by washing initially with dH₂O and then with PBS containing 0.05% Tween-20. The site of cytokine secretion was detected with a blotin-labeled anti-mouse cytokine antibody and horseradish peroxidase-conjugated streptavidin. The enzyme reaction was developed using 3-amino-9-ethylcarbazole (AEC) substrate reagent set (Sigma, Dorset, UK). Spot forming cell numbers were determined using a dissecting light microscope (Zeiss Stemi 2000) and expressed relative to 1x10⁶ cells plated. Statistical differences were established using ANOVA and Student-Newman-Keuls tests.

### Results:

Injection of OVA loaded microparticles induced greater numbers of OVA specific IL-4 secreting spleen cells than IFN-γ secreting cells, as detected using ELISPOT; a profile indicative of a Th2 type response, as shown in Figure 8. This trend was reversed if mice were injected with microparticles that contained both OVA and ssRNA. Indeed, injection of coencapsulated OVA and ssRNA engendered the greatest numbers of OVA specific IFN-γ secreting cells as compared with any other treatment group (P<0.05). Consistent with the dogma that CpG promotes Th1 responses, injection of OVA loaded CpG decorated microparticles or OVA in solution with CpG induced appreciable numbers of OVA specific IFN-γ secreting T-cells (P<0.05). The numbers of OVA specific IL-4 secreting cells in mice immunised with OVA loaded CpG decorated microparticles or OVA in solution with CpG was significantly lower (P<0.05) than in animals immunised with OVA loaded microparticles or OVA and poly-U loaded microparticles. Injection of a solution of OVA admixed with poly-U resulted in negligible numbers of OVA specific IL-4 and IFN-γ secreting splenocytes.

### Example 17:

### Analysis of cellular responses: Tetramer staining and FACS analysis of lymph node cells:

On day 35, mice immunised according to example 14 were killed and Inguinal lymph nodes draining the site of injection were removed and pooled for tetramer analysis. Single cell suspensions were prepared in supplemented RPMI-1640. Single cell suspensions of lymph node cells were stimulated with OVA (50 µg ml-1) for 72 hours at 37°C in a humidified 5% CO₂ environment. Following stimulation, cells were Isolated from the culture plates and stained using the iTAg^{™} MHC Class I Murine Tetramer - SA - PE kit (Beckman Coulter, Immunomics, France). In addition to tetramer staining, FITC coupled CD 3 (Pharmingen, BD Biosciences, UK) and Cy 5.5 coupled CD 8 (Pharmingen, BD Biosciences, UK) antibodies were also used to stain the cells. All staining and fixation procedures were carried out in accordance with the manufacturer's instructions. After fixing, cells were analysed on a BD FACScan flow cytometer. Corresponding isotype controls were used to establish quadrants and / or regions for analysis. Analyses were performed using Cell Quest Pro flow cytometry analysis software.

### Results:

Tetramer staining of lymph node cells from immunised mice revealed that injection of comicroencapsulated OVA and poly U can engender antigen specific CD8⁺ T-cells, as shown in Figure 9. Mice immunised with co-micorencapsulated OVA and Poly-U (Fig. 9A) had a greater number of CD8 cells with bound tetramer, as evidenced by the increased FL2 signal. Figure 9B shows a profile obtained from naive mice.

## Claims

1. A microparticle composition comprising
(a) a biodegradable polymer;
(b) an immunogenic single-stranded ribonucleic acid (ss-RNA);
(c) a biologically active macromolecule; and
(d) a stabilising agent;
wherein the biologically active macromolecule, the single-stranded ribonucleic acid (ss-RNA) and the stabilising agent are encapsulated inside and/or within the biodegradable polymer to provide a free outer surface of the microparticle.

2. A microparticle composition according to claim 1 wherein the biodegradable polymer is biocampatible and degrades in mammalian tissues.

3. A microparticle composition according to either of claims 1 or 2 wherein the biodegradable polymer is an aliphatic polyester.

4. A microparticle composition according to claims 1 to 3 wherein the biodegradable polymer is poly-lactide.

5. A microparticle composition according to any preceding claim wherein the immunogenic ss-RNA is capable of stimulating production of proinflammatory and/or anti-viral cytokines.

6. A microparticle composition according to claim 5 wherein the ss-RNA stimulates production of anti-viral cytokines

7. A microparticle composition according to claim 6 wherein the antiviral cytokines are INF-α and/or INF-β and/or IL-12.

8. A microparticle composition according to any of claims 1 to 7 wherein the ss-RNA is capable of stimulating the Toll-Like Receptors (TLR) of a host cell.

9. A microparticle composition according to claim 8 wherein the ss-RNA stimulates TLR-7 and/or TLR-8.

10. A microparticle composition according to any preceding claim wherein the ss-RNA has a sequence which is predominately rich in a single base

11. A microparticle composition according to claim 10 wherein the ss-RNA sequence is predominately made up of Guanidine and/ or Uracil.

12. A microparticle composition according to claims 10 or 11 wherein the ss-RNA is polyuridylic acid.

13. A microparticle composition according to any preceding claim wherein the biologically active macromolecule is an oligodeoxynucleotide or an antigen specific to a pathogen.

14. A microparticle composition according to claim 13 wherein the biologically active macromolecule is an antigen specific to a bacterial or viral pathogen.

15. A pharmaceutical composition according to claim 13 or14 wherein the biologically active macromolecule is recombinant Protective Antigen (rPA) of Bacillus anthracis.

16. A microparticle composition according to any preceding claim wherein the biologically active macromolecule is an antigen expressed on a tumour cell.

17. A microparticle composition according to any of the preceding claims wherein the stabilising agent is pharmaceutically acceptable compound which is capable of forming a complex with the ss-RNA.

18. A microparticle composition according to claim 17 wherein the stabilising agent is a cationic polymer or a cationic lipid.

19. A microparticle composition according to claims 17 or 18 wherein the stabilising agent is N-[1-(2,3-dioleoyloxy)propyl]-N,N,N- trimethylammonium chloride.

20. A microparticle composition according to any preceding claim wherein the composition has an overall net positive charge.

21. A microparticle composition according to claim 20 wherein the composition has a zeta potential in the range of 0 to 100 mV, preferably in the range of 20 to 80 mV and more preferably in the range of 30 to 60 mV.

22. A microparticle composition according to any preceding claim wherein the resulting microparticles have mean diameter in the range of 0.1 to 5 µm and more preferably in the range of 0.2 to 4 µm.

23. A microparticle composition according to claim 22 wherein the microparticles have mean diameter of about 1 µm.

24. A method of producing the microparticle composition of claims 1 to 23 comprising the steps of:
(a) preparing a solution of biodegradable polymer;
(b) adding to the solution of (a), a solution comprising an immunogenic ss-RNA and a biologically active macromolecule to form an emulsion
(c) Adding the emulsion from step (b) to a solution containing the stabilising agent to form a double emulsion;
(d) Removing the solvent; and
(e) Collecting the resulting microparticles.

25. A method according to claim 24 wherein the microparticles are further subjected to the step of lyophilisation.

26. A pharmaceutical composition comprising the microparticle composition according to any of claims 1 to 23 and a pharmaceutically acceptable adjuvant and/or excipient.

27. A pharmaceutical composition according to claim 26, for use in medicine.

28. Use of a pharmaceutical composition according to claim 26 in the manufacture of a medicament for the treatment of pathogenic infection.

29. Use of a pharmaceutical composition according to claim 26 in the manufacture of a medicament for stimulation of Toll-Like Receptors of a host cell.

30. Use of a pharmaceutical composition according to claim 26 in the manufacture of a medicament for the treatment of cancer.

## Patentansprüche

1. Mikropartikel-Zusammensetzung, die enthält:
(a) ein biologisch abbaubares Polymer,
(b) eine immunogene einzelsträngige Ribonucleinsäure (ss-RNA),
(c) ein biologisch aktives Makromolekül und
(d) ein Stabilisierungsmittel,
wobei das biologisch aktive Makromolekül, die einzelsträngige Ribonucleinsäure (ss-RNA) und das Stabilisierungsmittel im Inneren oder innerhalb des biologisch abbaubaren Polymers so eingekapselt sind, dass das Mikropartikel eine freie äußere Oberfläche aufweist.

2. Mikropartikel-Zusammensetzung nach Anspruch 1, bei der das biologisch abbaubare Polymer biokompatibel ist und in Säugergeweben abgebaut wird.

3. Mikropartikel-Zusammensetzung nach Anspruch 1 oder 2, bei der das biologisch abbaubare Polymer ein aliphatischer Polyester ist.

4. Mikropartikel-Zusammensetzung nach einem oder mehreren der Ansprüche 1 bis 3, bei der das biologisch abbaubare Polymer ein Polylactid ist.

5. Mikropartikel-Zusammensetzung nach einem oder mehreren der vorhergehenden Ansprüche, bei der die immunogene ss-RNA befähigt ist, die Produktion proinflammatorischer und /der antiviraler Cytokine zu stimulieren.

6. Mikropartikel-Zusammensetzung nach Anspruch 5, bei der die ss-RNA die Produktion antiviraler Cytokine stimuliert.

7. Mikropartikel-Zusammensetzung nach Anspruch 6, bei der die antiviralen Cytokine INF-α und/oder INF-β und/oder IL-12 sind.

8. Mikropartikel-Zusammensetzung nach einem oder mehreren der Ansprüche 1 bis 7, bei der die ss-RNA befähigt ist, die Toll-Like-Rezeptoren (TLR) einer Wirtszelle zu stimulieren.

9. Mikropartikel-Zusammensetzung nach Anspruch 8, bei der die ss-RNA TLR-7 und/oder TLR-8 stimuliert.

10. Mikropartikel-Zusammensetzung nach einem oder mehreren der vorhergehenden Ansprüche, bei der die ss-RNA eine Sequenz aufweist, die überwiegend reich an einer einzigen Base ist.

11. Mikropartikel-Zusammensetzung nach Anspruch 10, bei der die ss-RNA-Sequenz überwiegend aus Guanidin und/oder Uracil besteht.

12. Mikropartikel-Zusammensetzung nach Anspruch 10 oder 11, bei der die ss-RNA Polyuridylsäure ist.

13. Mikropartikel-Zusammensetzung nach einem oder mehreren der vorhergehenden Ansprüche, bei der das biologisch aktive Makromolekül ein Oligodesoxynucleotid oder ein für ein Pathogen spezifisches Antigen ist.

14. Mikropartikel-Zusammensetzung nach Anspruch 13, bei der das biologisch aktive Makromolekül ein für ein bakterielles oder virales Pathogen spezifisches Antigen ist.

15. Pharmazeutische Zusammensetzung nach Anspruch 13 oder 14, bei der das biologisch aktive Makromolekül das rekombinante Protective Antigen (rPA) von Bacillus anthracis ist.

16. Mikropartikel-Zusammensetzung nach einem oder mehreren der vorhergehenden Ansprüche, bei der das biologisch aktive Makromolekül ein auf einer Tumorzelle exprimiertes Antigen ist.

17. Mikropartikel-Zusammensetzung nach einem oder mehreren der vorhergehenden Ansprüche, bei der das Stabilisierungsmittel eine pharmazeutisch akzeptable Verbindung ist, die befähigt ist, einen Komplex mit der ss-RNA zu bilden.

18. Mikropartikel-Zusammensetzung nach Anspruch 17, bei der das Stabilisierungsmittel ein kationisches Polymer oder ein kationisches Lipid ist.

19. Mikropartikel-Zusammensetzung nach Anspruch 17 oder 18, bei der das Stabilisierungsmittel N-[1-(2,3-Dioleoyloxy)-propyl]-N,N,N-trimethylammoniumchlorid ist.

20. Mikropartikel-Zusammensetzung nach einem oder mehreren der vorhergehenden Ansprüche, wobei die Zusammensetzung eine positive Netto-Gesamtladung aufweist.

21. Mikropartikel-Zusammensetzung nach Anspruch 20, wobei die Zusammensetzung ein Zeta-Potential im Bereich von 0 bis 100 mV, vorzugsweise im Bereich von 20 bis 80 mV und noch bevorzugter im Bereich von 30 bis 60 mV aufweist.

22. Mikropartikel-Zusammensetzung nach einem oder mehreren der vorhergehenden Ansprüche, bei der die resultierenden Mikropartikel einen mittleren Durchmesser im Bereich von 0,1 bis 5 µm und noch bevorzugter im Bereich von 0,2 bis 4 µm aufweisen.

23. Mikropartikel-Zusammensetzung nach Anspruch 22, bei der die Mikropartikel einen mittleren Durchmesser von etwa 1 µm aufweisen.

24. Verfahren zur Herstellung der Mikropartikel-Zusammensetzung nach den Ansprüche 1 bis 23, das folgende Schritte umfasst:
(a) Herstellung einer Lösung eines biologisch abbaubaren Polymers;
(b) Zugabe einer Lösung, die eine immunogene ss-RNA und ein biologisch aktives Makromolekül enthält, zu der Lösung aus Schritt (a) unter Erzeugung einer Emulsion;
(c) Zugabe der Emulsion von Schritt (b) zu einer Lösung, die das Stabilisierungsmittel enthält, unter Bildung einer doppelten Emulsion;
(d) Entfernen des Lösungsmittels und
(e) Gewinnen der resultierenden Mikropartikel.

25. Verfahren nach Anspruch 24, bei dem die Mikropartikel ferner dem Schritt einer Lyophilisierung unterzogen werden.

26. Pharmazeutische Zusammensetzung, welche die Mikropartikel-Zusammensetzung nach einem der Ansprüche 1 bis 23 sowie einen pharmazeutisch akzeptablen Hilfsstoff und/oder ein pharmazeutisch akzeptables Excipiens enthält.

27. Pharmazeutische Zusammensetzung nach Anspruch 26 zur Anwendung in der Medizin.

28. Verwendung einer pharmazeutischen Zusammensetzung nach Anspruch 26 bei der Herstellung eines Arzneimittels zur Behandlung von durch Pathogene hervorgerufenen Infektionen.

29. Verwendung einer pharmazeutischen Zusammensetzung nach Anspruch 26 bei der Herstellung eines Arzneimittels zur Stimulation der Toll-Like-Rezeptoren einer Wirtszelle.

30. Verwendung einer pharmazeutischen Zusammensetzung nach Anspruch 26 bei der Herstellung eines Arzneimittels zur Behandlung von Krebs.

## Revendications

1. Composition de microparticules comprenant :
(a) un polymère biodégradable;
(b) un acide ribonucléique en simple brin immunogène (ARN sb) ;
(c) une macromolécule biologiquement active ; et
(d) un agent stabilisant ;
dans laquelle la macromolécule biologiquement active, l'acide ribonucléique en simple brin (ARN sb) et l'agent stabilisant sont encapsulés à l'intérieur du et/ou dans le polymère biodégradable pour fournir une surface extérieure libre de la microparticule.

2. Composition de microparticules selon la revendication 1, dans lequel, le polymère biodégradable est biocompatible et se dégrade dans les tissus de mammifère.

3. Composition de microparticules selon l'une des revendications 1 ou 2, dans laquelle le polymère biodégradable est un polyester aliphatique.

4. Composition de microparticules selon les revendications 1 à 3, dans laquelle le polymère biodégradable est un polylactide.

5. Composition de microparticules selon l'une quelconque des revendications précédentes, dans laquelle l'ARN sb immunogène est capable de stimuler la production de cytokines pro-inflammatoires et/ou antivirales.

6. Composition de microparticules selon la revendication 5 dans laquelle l'ARN sb stimule la production de cytokines antivirales.

7. Composition de microparticules selon la revendication 6, dans lequel les cytokines antivirales sont INF-□ et/ou INF-□ et/ou IL-12.

8. Composition de microparticules selon l'une quelconque des revendications 1 à 7, dans laquelle l'ARN sb est capable de stimuler des récepteurs de type Toll (TLR) d'une cellule hôte.

9. Composition de microparticules selon la revendication 8, dans laquelle l'ARN sb stimule le TLR-7 et/ou le TLR-8.

10. Composition de microparticules selon l'une quelconque des revendications précédentes, dans laquelle l'ARN sb a une séquence qui est de manière prédominante riche en une seule base.

11. Composition de microparticules selon la revendication 10, dans laquelle la séquence d'ARN sb est principalement composée de Guanidine et/ou d'Uracile.

12. Composition de microparticles selon la revendication 10 ou 11, dans laquelle l'ARN sb est de l'acide polyuridylique.

13. Composition de microparticules selon l'une quelconque des revendications précédentes, dans laquelle la macromolécule biologiquement active est un oligodésoxy-nucléotide ou un antigène spécifique à un pathogène.

14. Composition de microparticules selon la revendication 13, dans laquelle la macroparticule biologiquement active est un antigène spécifique à un pathogène bactérien ou viral.

15. Composition pharmaceutique selon les revendications 13 ou 14, dans laquelle la macromolécule biologiquement active est un antigène protecteur recombinant (APr) de Bacillus anthracis.

16. Composition de microparticules selon l'une quelconque des revendications précédentes, dans laquelle la macromolécule biologiquement active est un antigène exprimé sur une cellule tumorale.

17. Composition de microparticules selon l'une quelconque des revendications précédentes, dans laquelle l'agent stabilisant est un composé pharmaceutiquement acceptable qui est capable de former un complexe avec l'ARN sb.

18. Composition de microparticules selon la revendication 17, dans laquelle l'agent stabilisant est un polymère cationique ou un lipide cationique.

19. Composition de microparticules selon les revendications 17 ou 18, dans laquelle l'agent stabilisant est du chlorure de N-[1-(2,3-(dioléoyloxy)propyl]-N,N,N-triméthylammonium.

20. Composition de microparticules selon l'une quelconque des revendications précédentes, dans laquelle la composition a une charge positive nette globale.

21. Composition de microparticules selon la revendication 20, dans laquelle la composition a un potentiel zêta dans la plage de 0 à 100 mV, de préférence dans la plage de 20 à 80 mV et encore de préférence dans la plage de 30 à 60 mV.

22. Composition de microparticles selon l'une quelconque des revendications précédentes, dans laquelle les microparticules résultantes ont un diamètre moyen dans la plage de 0,1 à 5 µm et plus de préférence dans la plage de 0,2 et 4 µm.

23. Composition de microparticules selon la revendication 22
dans laquelle les microparticules ont un diamètre moyen d'environ 1 µm.

24. Procédé de fabrication de la composition de microparticules selon les revendications 1 à 23 comprenant les étapes de :
(a) préparation d'une solution de polymère biodégradable ;
(b) ajout à la solution (a) d'une solution comprenant un ARN sb immunogène et une macromolécule biologiquement active pour former une émulsion.
(c) ajout de l'émulsion de l'étape (b) à une solution contenant l'agent stabilisant pour former une double émulsion,
(d) enlèvement du solvant ; et
(e) récupération des microparticules résultantes.

25. Procédé selon la revendication 24, dans laquelle les microparticules sont en outre soumises à une étape de lyophilisation.

26. Composition pharmaceutique comprenant la composition de microparticules selon l'une quelconque des revendications 1 à 23 et un adjuvant et/ou un excipient acceptable sur le plan pharmaceutique.

27. Composition pharmaceutique selon la revendication 26 destinée à être utilisée en médecine.

28. Utilisation d'une composition pharmaceutique selon la revendication 26 dans la fabrication d'un médicament destiné au traitement d'une infection pathogène.

29. Utilisation d'une composition pharmaceutique selon la revendication 26 dans la fabrication d'un médicament destiné à la stimulation de récepteurs de type Toll d'une cellule hôte.

30. Utilisation d'une composition pharmaceutique selon la revendication 26 dans la fabrication d'un médicament destiné au traitement du cancer.
